# EUROPÄISCHE PATENTSCHRIFT

(11) **EP 0 647 614 B1**
(45) Veröffentlichungstag und Bekanntmachung des Hinweises auf die Patenterteilung: **04.06.1997**
(21) Anmeldenummer: 94114772.0
(22) Anmeldetag: 20.09.1994
(51) Int. Cl.: C07C 227/08

(54) **Verfahren zur Herstellung von 5-Fluoranthranilsäure**
Process for the préparation of 5-fluoro anthranilic acid
Procédé pour la préparation de l'acide 5-fluoro anthranilique

(30) Priorität: 08.10.1993 DE 4334431
(43) Veröffentlichungstag der Anmeldung: 12.04.1995
(73) Patentinhaber: HOECHST AKTIENGESELLSCHAFT, 65929 Frankfurt am Main (DE)
(72) Erfinder: Röhrscheid, Freimund, Dr., D-65779 Kelkheim (DE); Rapp, Jochen, Dr., D-60322 Frankfurt am Main (DE); Papenfuhs, Theodor, Dr., D-60433 Frankfurt am Main (DE)

(56) Entgegenhaltungen:
- EP-A- 0 551 632
- J. BIOL. CHEM., Bd.207, 1954 Seiten 411 - 420 B.E. VOLCANI ET AL. 'Action of some substituted Anthranilic Acids on Escheria Coli'

## Beschreibung

Die vorliegende Erfindung betrifft ein neues, vorteilhaftes Verfahren zur Herstellung von 5-Fluoranthranilsäure.

Es ist bekannt, 5-Fluoranthranilsäure durch Nitrierung von 3-Fluorbenzoesäure und anschließende Reduktion der gebildeten 5-Fluor-2-nitrobenzoesäure herzustellen (Rec. Trav. Chim. Pays-Bas 1914, 33, 336, Tetrahedron Lett. 1988, 29(40), 5105-8; J. Biol, Chem. 1954, 207, 411-2).

Bei dieser Synthese bildet sich im Verlauf der Nitrierung stets auch die isomere 3-Fluor-2-nitrobenzoesäure, die sich jedoch von der 5-Fluor-2-nitrobenzoesäure nicht abtrennen läßt. Durch die nachfolgende Reduktion entsteht aus diesem Produktgemisch, das etwa 2 - 3 % 3-Fluor-2-nitrobenzoesäure enthält, neben dem gewünschten Wertprodukt (5-Fluoranthranilsäure) im entsprechenden Umfange die 3-Fluoranthranilsäure, die sich allerdings nicht von der 5-Fluoranthranilsäure abtrennen läßt.

Die DE-OS 4 200 512 beschreibt die Herstellung von Halogenanthranilsäuren, wobei eine entsprechende Halogen-2-brombenzoesäure mittels wäßrigem Ammoniak und in Gegenwart eines Kupferkatalysators umgesetzt wird. Das beanspruchte Verfahren umfaßt zwar auch eine Umsetzung von 5-Fluor-2-brombenzoesäure zu 5-Fluoranthranilsäure, belegt aber wird diese Herstellung weder durch ein Beispiel noch durch Angaben hinsichtlich erzielbarer Ausbeuten und Reinheiten. Zudem gestaltet sich diese Synthese durch den bevorzugten Einsatz zusätzlicher Lösungsmittel, und durch die Verwendung von Kupferverbindungen als Katalysator, deren nachträgliche Abtrennung aus dem Reaktionsprodukt und der Mutterlauge technisch sehr aufwendig ist, recht schwierig. Die Entfernung der Kupfersalze aus den Abwässern erweist sich als besonders problematisch, da die Kupfersalze in komplexierter Form vorliegen. Eine Angabe, wie diese Kupfersalze aus den Abwässern zu entfernen sind, ist der DE-OS1 4 200 512 nicht zu entnehmen.

Die für die Umsetzung mit Ammoniak benötigte 5-Fluor-2-brombenzoesäure läßt sich durch Oxidation von 5-Fluor-2-bromtoluol mittels Permanganat herstellen (J. Indian Chem. Soc. 1944, 21, 112-4; J. Org. Chem. 1988, 53(2), 345-52). Dieses Verfahren liefert allerdings das gewünschte Wertprodukt lediglich in einer Ausbeute von 70 %. Zudem fallen, bedingt durch die Oxidation mit Permanganat, Rückstände an, die Mangan in erheblichen Mengen enthalten. Derartige Rückstände sind schwierig und nur unter großem Aufwand zu entsorgen.

Überträgt man die Lehre der DE-OS 4 200 512 auf die Herstellung von 5-Fluoranthranilsäure, so zeigt es sich, daß die Umsetzung von 5-Fluor-2-brombenzoesäure mittels wäßrigem Ammoniak in Gegenwart eines Kupferkatalysators nicht zu dem gewünschten reinen Wertprodukt führt. Vielmehr liefert dieses Verfahren eine stark durch 4,4'-Difluordiphenylamin-2,2'-dicarbonsäure verunreinigte 5-Fluoranthranilsäure, die sich jedoch mit einem technisch vertretbaren Aufwandes nicht mehr reinigen läßt. Der entsprechende Versuchsbefund ist Vergleichsbeispiel 1 zu entnehmen.

Da 5-Fluoranthranilsäure ein wertvolles Vorprodukt zur Herstellung von Pharmazeutika (Cz.P 159 570), Herbiciden (US 3 905 800; EP 0 109 575; US 4 388 472), Pflanzenwachstumsregulatoren (FR 2 541 288) und Fungiciden vom Chinazolintyp (US 4 824 469) darstellt, werden an die Reinheit der als Ausgangsprodukt verwendeten 5-Fluoranthranilsäure sehr hohe Anforderungen gestellt.
Es besteht daher ein erhebliches Interesse, 5-Fluoranthranilsäure auf technisch einfache Weise, in hoher Ausbeute und hoher Reinheit zugänglich zu machen. Zugleich sollen die vorstehend beschriebenen Nachteile vermieden werden.

Gelöst wird diese Aufgabe durch ein neues Verfahren zur Herstellung von 5-Fluoranthranilsäure. Es ist dadurch gekennzeichnet, daß man 5-Fluor-2-bromtoluol in Anwesenheit eines Katalysators und eines sauren Lösungsmittels mit Sauerstoff oder einem Sauerstoff enthaltenden Gas bei 80 - 220°C umsetzt, die gebildete 5-Fluor-2-brombenzoesäure abtrennt und mit Ammoniak bei 70 - 180°C umsetzt.

Das erfindungsgemäße Verfahren umgeht die Schwierigkeiten der Permanganatoxidation, indem man technisch verfügbares 5-Fluor-2-bromtoluol auf einfache Weise durch katalytische Oxidation mittels Sauerstoff oder eines Sauerstoff enthaltenden Gases mit hoher Ausbeute und sehr guter Reinheit zu 5-Fluor-2-brombenzoesäure umsetzt, ohne einen Anfall manganhaltiger Rückstände in Kauf nehmen zu müssen.

Ferner wurde überraschenderweise gefunden, daß sich 5-Fluor-2-brombenzoesäure mit Ammoniak, insbesondere wäßrigem Ammoniak, ohne Zusatz eines organischen Lösungsmittels und ohne Zusatz eines Kupferkatalyators umsetzen läßt, wobei die Umsetzung nicht nur problemlos verläuft, sondern auch das gewünschte Wertprodukt unter Vermeidung unerwünschter Nebenreaktionen in sehr hoher Reinheit liefert. Die nach diesem Verfahren hergestellte 5-Fluoranthranilsäure fällt in einer Reinheit an, wie sie zur Herstellung der vorstehend erwähnten biologisch aktiven Wirkstoffe ohne Einschränkung verwendet werden kann.

Man setzt 5-Fluor-2-bromtoluol mit Sauerstoff oder einem Sauerstoff enthaltenden Gas in Anwesenheit eines Übergangsmetallionen enthaltenden Katalysators um.
Gut geeignet als Katalysator ist eine Verbindung, die Kobaltionen enthält.

Besonders einfach läßt sich das Verfahren gestalten, indem man einen Katalysator, der neben Kobaltionen auch Manganionen enthält, verwendet. Verwendet man einen Katalysator, der zusätzlich zu den Übergangsmetallionen noch Bromidionen enthält, so beeinflußt dies die Umsetzung günstig.
Es empfiehlt sich, einen Katalysator zu verwenden, der Übergangsmetallionen und Bromidionen im Verhältnis 1:(0,01 bis 2), insbesondere 1:(0,1 bis 1) bevorzugt 1:(0,2 bis 0,7) enthält.
Verwendet man einen Kobaltionen- und Manganionen enthaltenden Katalysator, so empfiehlt es sich, die Kobaltionen und Manganionen in einem Verhältnis von 1:(0,01 bis 3) insbesondere 1:(0,05 bis 1) einzusetzen.

Im allgemeinen genügt es, den Katalysator entsprechend 0,002 bis 0,1, insbesondere 0,003 bis 0,03, bevorzugt 0,005 bis 0,015 Mol Übergangsmetallionen, bezogen auf 1 Mol 5-Fluor-2-bromtoluol einzusetzen.

Die Umsetzung wird in Anwesenheit eines sauren Lösungsmittels durchgeführt. Üblicherweise verwendet man eine aliphatische Carbonsäure mit 1 - 8, insbesondere 2 - 6 Kohlenstoffatomen als saures Lösungsmittel. Es lassen sich jedoch auch Mischungen dieser Carbonsäuren verwenden.
Gut geeignet als saures Lösungsmittel sind Essigsäure, Propionsäure, n-Buttersäure oder Iso-buttersäure oder Mischungen derselben. Besonders einfach gestaltet sich das Verfahren, wenn man Essigsäure und/oder Propionsäure als saures Lösungsmittel einsetzt. In einer Reihe von Fällen erweist sich Essigsäure als besonders geeignetes saures Lösungsmittel.
Üblicherweise setzt man das saure Lösungsmittel in wasserfreier Form ein. Während der Umsetzung von 5-Fluor-2-bromtoluol mit Sauerstoff oder dem Sauerstoff enthaltenden Gas ist darauf zu achten, daß das Reaktionsgemisch nicht mehr als 15 Gew.-% Wasser enthält. Ein höherer Wassergehalt verhindert zwar die Umsetzung nicht, führt aber zu einer Herabsetzung der Reaktionsgeschwindigkeit. Besonders günstig ist es, während der Umsetzung von 5-Fluor-2-bromtoluol den Wassergehalt des Reaktionsgemisches auf einen Wert nicht mehr als 5 Gew.-% zu begrenzen.
Der Wassergehalt des Reaktionsgemisches läßt sich dadurch steuern, daß man Wasser aus dem Reaktionsgemisch kontinuierlich oder diskontinuierlich abdestilliert.

Die Oxidation von 5-Fluor-2-bromtoluol läßt sich mittels reinem Sauerstoff oder mittels eines Sauerstoff enthaltenden Gases durchführen. Besonders gut geeignet als Sauerstoff enthaltendes Gas ist Luft, insbesondere trockene Luft. Wie bereits zuvor erwähnt, führt man die Oxidation üblicherweise bei einer Temperatur von 80 - 220°C durch.
In einer Vielzahl von Fällen genügt es, die Oxidation bei 130 - 180, insbesondere 135 - 160°C durchzuführen.

Nach Beendigung der Oxidation trennt man die gebildete 5-Fluor-2-brombenzoesäure, beispielsweise durch Filtration oder Zentrifugieren, ab und befreit das anfallende Filtrat destillativ vom Wasser, das sich einerseits während der Reaktion bildet, andererseits aber durch Verwendung wasserhaltiger Einsatzstoffe in das Reaktionsprodukt gelangen kann.

Die Oxidation läßt sich günstig beeinflussen, wenn man einen Sauerstoffpartialdruck von mindestens 0,15, insbesondere 0,25, bevorzugt 0,4 MPa, gemessen an der Eintrittsstelle des Sauerstoffs in die flüssige Phase, anwendet. Die Übergangsmetallionen werden bevorzugt in Form von Carbonsäuresalzen zugefügt. Die Bromidionen können in Form einer HBr-Lösung oder als Kaliumbromid, Natriumbromid, Kobalt- oder Manganbromid zugegeben werden.

Die 5-Fluor-2-brombenzoesäure kristallisiert, gegebenenfalls unter Abkühlung, aus dem bei der Oxidation anfallenden Reaktionsgemisch und wird üblicherweise durch Filtration abgetrennt. Da sich im Fitrat jedoch erhebliche Mengen an gelöstem Wertprodukt und der Katalysator befinden, empfiehlt es sich, aus dem Filtrat das enthaltene Wasser an einer Kolonne abzudestillieren und die als Sumpf anfallende, den Katalysator und das saure Lösungsmittel enthaltende aufkonzentrierte Mutterlauge wieder als Reaktionsmedium für die Oxidation zu verwenden. Die Mutterlauge läßt sich ohne weiteres bis zu 10 mal und mehr in die Oxidation wieder einsetzen, bevor sie durch Destillation in wiederverwendbares saures Lösungsmittel und einen deponierbaren Rückstand getrennt werden muß.

Ein weiterer großer Vorteil des erfindungsgemäßen Verfahrens gegenüber dem Stand der Technik besteht darin, daß das vom Wasser befreite Filtrat (Mutterlauge) erneut als Reaktionsmedium für die Oxidation eingesetzt werden kann, während bei der Permangantoxidation lediglich ein stark manganhaltiges Abwasser, das schwer zu entsorgen ist, anfällt.

Die 5-Fluor-2-brombenzoesäure wird anschließend ohne Anwesenheit eines Katalysators, mit Ammoniak umgesetzt. Besonders einfach ist es, Ammoniak in Form einer wäßrigen Lösung zu verwenden. Üblicherweise verwendet man eine 20 - 50, insbesondere 25 - 33 Gew.-% Ammoniak enthaltende wäßrige Lösung.

Üblicherweise führt man, wie bereits zuvor erwähnt, die Umsetzung mit Ammoniak bei 70 - 180°C durch. In einer Vielzahl von Fällen genügt es, die Umsetzung mit Ammoniak bei 90 - 150, insbesondere 100 - 130°C durchzuführen.
Die Umsetzung läßt sich bei Normaldruck durchführen. Das Verfahren vereinfacht sich jedoch, wenn man unter Druck arbeitet. Im allgemeinen genügt es, bei einem Druck von 0,2 - 2,5, insbesondere 0,3 - 1,5, bevorzugt 0,5 - 1,2 MPa zu arbeiten. Es ist aber auch möglich, bei höheren Drücken zu arbeiten, was jedoch in der Regel die Verwendung geeigneter Druckapparaturen erforderlich macht.

Im allgemeinen setzt man Ammoniak im Überschuß, bezogen auf die für die Umsetzung erforderliche stöchiometrische Menge von 3 Moläquivalenten je Mol 5-Fluor-2-brombenzoesäure, zu. In den meisten Fällen erweist sich ein Verhältnis von 8 - 10 Mol Ammoniak pro Mol 5-Fluor-2-brombenzoesäure als ausreichend. Der stöchiometrisch erforderliche Ammoniakbedarf läßt sich um ein Drittel senken, wenn man die 5-Fluor-2-brombenzoesäure in Form ihrer Salze, insbesondere Alkalisalze oder deren wäßrige Lösungen einsetzt.

Setzt man wäßrige Lösungen von 5-Fluor-2-brombenzoesäure-alkalisalzen ein, so ist darauf zu achten, daß die Ammoniak-Konzentration in der Reaktionsmischung 20 - 50, bevorzugt 25 - 33 Gew.-% beträgt. Dies kann die Verwendung von höher konzentrierten wäßrigen Ammoniaklösungen oder reinem Ammoniak notwendig machen.
Die Umsetzung kann man absatzweise durchführen, indem man alle Reaktionspartner bei Raumtemperatur vorlegt und anschließend die Reaktion durchführt. Recht günstig ist eine Arbeitsweise, eine wäßrige, auf Reaktionstemperatur vorgeheizte Ammoniaklösung vorzulegen und eine Lösung eines Ammonium- und/oder Alkalisalzes der 5-Fluor-2-brombenzoesäure in Wasser kontinuierlich zuzupumpen, wobei durch Heizen die Reaktionstemperatur eingehalten wird. Nach Abschluß der Umsetzung, Abkühlen und Entspannen werden dem Reaktionsgemisch je Mol 5-Fluoranthranilsäure 2 Mol Äquivalente Alkalihydroxid, bei Einsatz von Alkalisalzen der 5-Fluor-2-brombenzoesäure nur 1 Mol Äquivalent Alkalihydroxid, zugegeben und überschüssiges Ammoniak unter Rückgewinnung einer wiedereinsetzbaren wäßrigen Ammoniaklösung abdestilliert.
Die anfallende Alkalisalzlösung der 5-Fluoranthranilsäure kann durch Neutralisation mit wäßriger Mineralsäure, beispielsweise wäßriger Salz- oder Schwefelsäure, gefällt und die dabei ausfallende reine 5-Fluoranthranilsäure durch Filtration, Waschen mit ionenfreiem Wasser und Trocknen bei erhöhter Temperatur isoliert werden. Eine 5-Fluoranthranilsäure, die völlig frei von nachweisbaren Verunreinigungen ist, läßt sich erhalten, indem man ihre saure Lösung mit Aktivkohle behandelt. Hierzu kann man eine bereits isolierte 5-Fluoranthranilsäure verwenden, besonders einfach ist es jedoch, die nach der Ammoniak-Rückgewinnung vorliegende wäßrige Alkalisalzlösung der 5-Fluoranthranilsäure mit wäßriger Mineralsäure, beispielsweise wäßriger Salz- oder Schwefelsäure, auf pH = 0,5 - 1 einzustellen, die Lösung mit Aktivkohle zu behandeln und das Filtrat mit Alkalihydroxid zu neutralisieren. Die anschließend nach Filtration, Wäsche und Trocknung anfallende 5-Fluoranthranilsäure ist von hoher Reinheit (analysenrein).

Die nachfolgenden Beispiele beschreiben die Erfindung, ohne sie zu beschränken.

### Experimenteller Teil

### Beispiel 1 (Startansatz)

Eine Lösung aus

| | | |
|---|---|---|
| 2,99 | Teilen | Co(OAc)₂ · 4H₂O |
| 0,98 | Teilen | Mn(OAc)₂ · 4H₂O |
| 0,405 | Teilen | HBr |
| 302,4 | Teilen | 5-Fluor-2-bromtoluol |
| 330,0 | Teilen | Eisessig |

wird in einem Autoklav vorgelegt und unter Rühren bei 16 bar N₂-Druck auf 145°C geheizt. Dann wird Luft (16 bar) eingeleitet, worauf sofort unter O₂-Verbrauch die stark exotherme Reaktion einsetzt. Die Temperatur wird auf 160°C eingestellt. Nach 70 Minuten ist die Reaktion beendet. Die homogene Reaktionslösung wird bei 100°C aus dem Autoklaven entnommen und unter Rühren abgekühlt.

Das ausgefallene Kristallisat wird über eine Nutsche scharf abgesaugt, mit 5 x 25 Teilen 90 %iger wäßriger Essigsäure gewaschen und getrocknet.

| | |
|---|---|
| Ausbeute | 275,8 Teile (78,7 d.Th.) 5-Fluor-2-brombenzoesäure |
| Festpunkt | 154 - 155°C |
| Reinheit | > 99 % |

Das Einengen der Mutterlauge auf 1/4 des ursprünglichen Volumens liefert zusätzlich 35,0 Teile (ca. 10 % d.Th.) 5-Fluor-2-brombenzoesäure.

### Beispiel 2 - 7 (Folgeansätze)

Zur Mutterlauge des jeweils vorhergehenden Ansatzes, durchgeführt analog Beispiel 1, aus der das Wasser und ein Teil der Essigsäure jeweils abdestilliert wurde, so daß 335 Teile entwässerte Mutterlauge resultieren, werden

| | | |
|---|---|---|
| 0,125 | Teile | Co(OAc)₂ 4H₂O, |
| 0,049 | Teile | Mn(OAc)₂ 4H₂O und |
| 302,4 | Teile | 5-Fluor-2-bromtoluol |

zugefügt und diese Lösung jeweils wie in Beispiel 1 beschrieben oxidiert und aufgearbeitet. Die resultierende Mutterlauge dient nach Entwässerung und Aufkonzentrierung auf 335 Teile jeweils als Vorlage für den nachfolgenden Ansatz. Insgesamt werden 5 Mutterlaugen-Rückführungen durchgeführt. Dabei wird in allen Ansätzen eine qualitativ gleichwertige 5-Fluor-2-brombenzosäure (Reinheit > 99 %) reproduziert.

| | |
|---|---|
| Ausbeute | (Durchschnitt Beispiel 2 - 7): 326,8 Teile (93,3 % d.Th.) 5(Fluor-2-brombenzossäure |
| Festpunkt | 152 - 154°C |

### Beispiel 8

Eine Mischung aus 219 Teilen 5-Fluor-2-brombenzosäure und 680 Teilen 25 %igem wäßrigem Ammoniak wird im Autoklaven 2 Stunden auf 140°C erhitzt, wobei sich ein Druck von 1,0 MPa einstellt. Anschließend wird auf 30°C abgekühlt, entspannt und nach Zusatz von 160 Teilen 50 %iger Natronlauge das überschüssige Ammoniak abdestilliert. Als Destillat werden 550 Teile 25 %iges wäßriges Ammoniak erhalten, die im nächsten Ansatz wieder eingesetzt werden.

Die resultierende Lösung des 5-Fluoranthranilsäure-Natriumsalzes wird mit 670 Teilen 15 %iger Salzsäure auf pH 0,5 eingestellt und nach Zusatz von 20 Teilen Aktivkohle bei 95°C geklärt.

Man stellt das Filtrat mit 269 Teilen 35 %iger Natronlauge auf pH 3,5 zurück, kühlt auf 20°C und saugt den ausgefallenen Niederschlag ab. Nach Waschen mit ionenfreiem Wasser und Trocknen im Vakuum bei 100°C erhält man 147 Teile 5-Fluoranthranilsäure vom Fp. 182,5-182,7°C mit einem Reingehalt von 100 % (HPLC). Dies entspricht einer Ausbeute von 95,0 % der Theorie, bezogen auf eingesetzte 5-Fluor-2-brombenzoesäure. Führt man die Reaktion bei 110°C 5 Stunden durch, so erhält man ein identisches Ergebnis.

### Beispiel 9

510 Teile 30 %iges wäßriges Ammoniak werden im geschlossenen Autoklaven auf 130°C erhitzt, wobei sich ein Druck von 0,9 MPa einstellt. Dann wird im Verlaufe von 2 Stunden eine wäßrige 5-Fluor-2-brombenzoesäure-Natriumsalzlösung, hergestellt aus 219 Teilen 5-Fluor-2-brombenzoesäure und 240 Teilen 16,7 %iger wäßriger Natronlauge, kontinuierlich mittels einer Druckdosierpumpe zugepumpt. Die Reaktionstemperatur wird durch leichtes Heizen während des gesamten Dosiervorganges bei 130°C gehalten und nach Dosierende 30 Minuten bei dieser Temperatur nachgerührt. Der Druck steigt während der Reaktion nur unwesentlich auf 0,95 MPa an.

Anschließend wird abgekühlt, entspannt und nach Zusatz von 120 Teilen 33 %iger Natronlauge das überschüssige Ammoniak abdestilliert, wobei 500 Teile wieder einsetzbares 25 %iges wäßriges Ammoniak als Destillat anfallen.

Nach Zusatz von 1500 Teilen Wasser wird die 5-Fluoranthranilsäure-Natriumsalzlösung (pH 9,5) mit 30 %iger Salzsäure auf pH 3,8 eingestellt, wobei die 5-Fluoranthranilsäure in farblosen Kristallen ausfällt. Nach Abkühlen auf 10°C wird das Kristallisat abgesaugt, mit ionenfreiem Wasser salzfrei und neutral gewaschen und bei 100°C im Vakuum bis zur Gewichtskonstanz getrocknet.

Man erhält 151 Teile 5-Fluoranthranilsäure vom Fp. 182 - 182,5°C mit einem Reingehalt von 99,8 % (HPLC), was einer Ausbeute von 97,2 % der Theorie, bezogen auf 5-Fluor-2-brombenzoesäure, entspricht.

Setzt man an Stelle der 5-Fluor-2-brombenzosäure-Natriumsalzlösung aliquote Teile Kalium- oder Ammoniumsalzlösung, hergestellt durch Auflösen von 219 Teilen 5-Fluor-2-brombenzoesäure in 256,1 Teilen 21,9 %iger wäßriger Kalilauge bzw. 217 Teilen 7,9 %iger wäßriger Ammoniaklösung, als Dosiermedium ein und verfährt sonst in der angegebenen Weise, so erhält man die 5-Fluoranthranilsäure in identischer Ausbeute und Qualität.

Ein vergleichbares Ergebnis wird auch erhalten, wenn man die Dosierung auf 4 Stunden erhöht und die Reaktion bei 120°C durchführt.

### Beispiel 10

Wird nach Beispiel 9 verfahren, nach dem Abdestillieren des überschüssigen Ammoniaks aber nach Ansäuern der 5-Fluoranthranilatlösung bis pH 0,5 gemäß Beispiel 8 eine Kohleklärung durchgeführt und dann, wie in Beispiel 8 angegeben, aufgearbeitet, so erhält man 152 Teile 5-Fluoranthranilsäure vom Fp. 182,6 - 182,7°C mit einem Reingehalt von 100 % (HPLC), was einer Ausbeute von 98,1 % der Theorie, bezogen auf 5-Fluor-2-brombenzoesäure, entspricht.

### Vergleichsbeispiel 1

Durchführung der Aminierung von 5-Fluor-2-brombenzoesäure gemäß Beispiel 5 (in Verbindung mit Beispiel 1) der DOS 42,00,512.

In einem Glaskolben legt man 1,3 Teile Kupfer(I)oxid in 69 Teilen einer wäßrigen 25 %igen Ammoniaklösung unter Stickstoff vor. Dazu gibt man unter Rühren bei 25°C innerhalb von 5 Minuten eine Lösung von 21,9 Teilen 5-Fluor-2-brombenzosäure in 18 Teilen 25 %iger Ammoniaklösung und 60 Vol.-Teile Essigsäureethylester zu.

Die Reaktion wird bis zum Ende unter Stickstoff durchgeführt. Nach Zugabe der Ammoniumbenzoatlösung steigt die Reaktionstemperatur auf ca. 40°C. Die anfangs rötliche Kupfer(I)oxidsuspension geht in eine tiefblaue Lösung über. Nach 1 Stunde Rühren bei Raumtemperatur ist die Reaktion beendet. Zum Komplexieren des Kupfers gibt man 5,3 Teile Ethylendiamintetraessigsäure in den Ansatz, säuert mit Salzsäure auf pH 3,1 an und destilliert den Essigester ab. Das Produkt wird bei Raumtemperatur isoliert.

Nach Filtration, Wäsche mit ionenfreiem Wasser und Trocknen im Vakuum bei 100°C erhält man 14 Teile 5-Fluoranthranilsäure vom Fp. 177 - 177,5°C mit einem Reingehalt von 92,7 % (HPLC), was einer Ausbeute von 83,7 % der Theorie, bezogen auf eingesetzte 5-Fluor-2-brombenzoesäure, entspricht.

Als Hauptverunreinigung wurde durch Vergleich mit einer unabhängig durch stöchiometrische Umsetzung von 5-Fluor-2-brombenzoesäure mit 5-Fluoranthranilsäure und Ätznatron in Dimethylacetamid synthetisierten Reinsubstanz (RG durch HPLC: 99,8 %, Fp. 281 - 283°C) 4,4'-Difluordiphenylamin-2,2'-dicarbonsäure identifiziert und zu 7,1 % (HPLC) bestimmt.

Eine so verunreinigte 5-Fluoranthranilsäure ist zur Herstellung der obengenannten Pharma- und Agrowirkstoffe ungeeignet. Eine Reinigung durch Umkristallisation oder Umfällung gelingt nicht.

### Vergleichsbeispiel 2

Durchführung der Aminierung nach der erfindungsgemäßen Verfahrensweise, aber in Gegenwart von Cu-Salz.

Das Beispiel 9 wird exakt wiederholt mit dem einzigen Unterschied, daß zur vorgelegten Ammoniaklösung 1 Teil CuCl zugesetzt wird. Nach Zudosieren der 5-Fluor-2-brombenzoatlösung und Aufarbeitung werden 144 Teile 5-Fluoranthranilsäure vom Fp. 176,5 - 177,5°C mit einem Reingehalt von 91,9 % (HPLC) erhalten, was einer Ausbeute von 85,4 % der Theorie, bezogen auf eingesetzte 5-Fluor-2-brombenzoesäure, entspricht.

Analog Vergleichsbeispiel 1 wurde als Hauptverunreinigung 4,4'-Difluordiphenylamin-2,2'-dicarbonsäure identifiziert und zu 7,6 % bestimmt. Eine Reinigung durch Umkristallisation oder Umfällen zu einer zur Herstellung der vorgenannten Pharma- und Agrowirkstoffe geeigneten Qualität gelingt nicht.

## Patentansprüche

1. Verfahren zur Herstellung von 5-Fluoranthranilsäure, dadurch gekennzeichnet, daß man 5-Fluor-2-bromtoluol in Anwesenheit eines Katalysators und eines sauren Lösungsmittels mit Sauerstoff oder einem Sauerstoff enthaltenden Gas bei 80 bis 220°C umsetzt, die gebildete 5-Fluor-2-brombenzoesäure abtrennt und mit Ammoniak bei 70 bis 180°C umsetzt.

2. Verfahren nach Anspruch 1, dadurch gekennzeichnet, daß der Katalysator Übergangsmetallionen enthält.

3. Verfahren nach Anspruch 1 oder 2, dadurch gekennzeichnet, daß der Katalysator Kobaltionen enthält.

4. Verfahren nach einem oder mehreren der Ansprüche 1 bis 3, dadurch gekennzeichnet, daß der Katalysator Kobalt- und Manganionen enthält.

5. Verfahren nach einen oder mehreren der Ansprüche 1 bis 4, dadurch gekennzeichnet, daß der Katalysator zusätzlich zu den Übergangsmetallionen Bromidionen enthält.

6. Verfahren nach einem oder mehreren der Ansprüche 1 bis 5, dadurch gekennzeichnet, daß der Katalysator Übergangsmetallionen und Bromidionen im Verhältnis 1:(0,01 bis 2), insbesondere 1:(0,1 bis 1) bevorzugt 1:(0,2 bis 0,7), enthält.

7. Verfahren nach einem oder mehreren der Ansprüche 1 bis 6, dadurch gekennzeichnet, daß der Katalysator Kobalt- und Manganionen im Verhältnis 1:(0,01 bis 3), insbesondere 1:(0,05 bis 1) enthält.

8. Verfahren nach einem oder mehreren der Ansprüche 1 bis 7, dadurch gekennzeichnet, daß man den Katalysator entsprechend 0,002 bis 0,1, insbesondere 0,003 bis 0,03, bevorzugt 0,005 bis 0,015 Mol Übergangsmetallionen, bezogen auf 1 Mol 5-Fluor-2-bromtoluol einsetzt.

9. Verfahren nach einem oder mehreren der Ansprüche 1 bis 8, dadurch gekennzeichnet, daß man als saures Lösungsmittel eine aliphatische Carbonsäure mit 1 bis 8, insbesondere 2 bis 6 Kohlenstoffatomen einsetzt.

10. Verfahren nach einem oder mehreren der Ansprüche 1 bis 9, dadurch gekennzeichnet, daß man Essigsäure, Propionsäure, n-Buttersäure oder i-Buttersäure oder Mischungen derselben als saures Lösungsmittel einsetzt.

11. Verfahren nach einem oder mehreren der Ansprüche 1 bis 10, dadurch gekennzeichnet, daß man Essigsäure und/oder Propionsäure, insbesondere Essigsäure als saures Lösungsmittel einsetzt.

12. Verfahren nach einem oder mehreren der Ansprüche 1 bis 11, dadurch gekennzeichnet, daß das saure Lösungsmittel in wasserfreier Form eingesetzt wird.

13. Verfahren nach einem oder mehreren der Ansprüche 1 bis 12, dadurch gekennzeichnet, daß während der Umsetzung mit Sauerstoff oder einem Sauerstoff enthaltendem Gas das Reaktionsgemisch nicht mehr als 15 Gew.-% Wasser enthält.

14. Verfahren nach einem oder mehreren der Ansprüche 1 bis 13, dadurch gekennzeichnet, daß während der Umsetzung mit Sauerstoff oder einem Sauerstoff enthaltenden Gas das Reaktionsgemisch nicht mehr als 5 Gew.-% Wasser enthält.

15. Verfahren nach einem oder mehreren der Ansprüche 1 bis 14, dadurch gekennzeichnet, daß man als Sauerstoff enthaltendes Gas Luft, insbesondere trockene Luft einsetzt.

16. Verfahren nach einem oder mehreren der Ansprüche der Ansprüche 1 bis 15, dadurch gekennzeichnet, daß man die Umsetzung mit Sauerstoff oder einem Sauerstoff enthaltenden Gas bei 130 bis 180, insbesondere 135 bis 160°C durchführt.

17. Verfahren nach einem oder mehreren der Ansprüche 1 bis 16, dadurch gekennzeichnet, daß man einen Sauerstoffpartialdruck von mindestens 0,15 MPa, gemessen an der Eintrittsstelle in die flüssige Phase, anwendet.

18. Verfahren nach einem oder mehreren der Ansprüche 1 bis 17, dadurch gekennzeichnet, daß man einen Sauerstoffpartialdruck von mindestens 0,25, insbesondere 0,4 MPa, gemessen an der Eintrittsstelle in die flüssige Phase, anwendet.

19. Verfahren nach einem oder mehreren der Ansprüche 1 bis 18, dadurch gekennzeichnet, daß man die gebildete 5-Fluor-2-brombenzoesäure durch Filtration abtrennt, das anfallende Filtrat von Wasser destillativ befreit und als Reaktionsmedium erneut einsetzt.

20. Verfahren nach einem oder mehreren der Ansprüche 1 bis 19, dadurch gekennzeichnet, daß man die 5-Fluor-2-brombenzoesäure mit einer wäßrigen Ammoniaklösung umsetzt.

21. Verfahren nach einem oder mehreren der Ansprüche 1 bis 20, dadurch gekennzeichnet, daß man die 5-Fluor-2-brombenzoesäure mit einer 20 bis 50, insbesondere 25 bis 33 Gew.-% Ammoniak enthaltenden, wäßrigen Lösung umsetzt.

22. Verfahren nach einem oder mehreren der Ansprüche 1 bis 21, dadurch gekennzeichnet, daß man die Umsetzung mit Ammoniak bei 90 bis 150, insbesondere 100 bis 130°C durchführt.

23. Verfahren nach einem oder mehreren der Ansprüche 1 bis 22, dadurch gekennzeichnet, daß man die Umsetzung mit Ammoniak bei einem Druck von 0,2 bis 2,5, insbesondere 0,3 bis 1,5 MPa, bevorzugt 0,5 bis 1,2 MPa durchführt.

24. Verfahren nach einem oder mehreren der Ansprüche 1 bis 23, dadurch gekennzeichnet, daß man 5-Fluor-2-brombenzoesäure in Form einer Lösung eines Ammonium- und/oder Alkalisalzes der 5-Fluor-2-brombenzoesäure in Wasser kontinuierlich einer Ammoniaklösung zuleitet.

25. Verfahren nach einem der mehreren der Ansprüche 1 bis 24, dadurch gekennzeichnet, daß man eine saure Lösung von 5-Fluoranthranilsäure in Wasser mit Aktivkohle behandelt, die Aktivkohle abfiltriert und aus dem Filtrat durch Zugabe von Alkali 5-Fluoranthranilsäure abscheidet.

## Claims

1. A process for the preparation of 5-fluoroanthranilic acid, which comprises reacting 5-fluoro-2-bromotoluene in the presence of a catalyst and of an acidic solvent with oxygen or an oxygen-containing gas at from 80 to 220°C, separating off the 5-fluoro-2-bromobenzoic acid formed and reacting it with ammonia at from 70 to 180°C.

2. The process as claimed in claim 1, wherein the catalyst contains transition metal ions.

3. The process as claimed in claim 1 or 2, wherein the catalyst contains cobalt ions.

4. The process as claimed in one or more of claims 1 to 3, wherein the catalyst contains cobalt ions and manganese ions.

5. The process as claimed in one or more of claims 1 to 4, wherein the catalyst contains bromide ions in addition to the transition metal ions.

6. The process as claimed in one or more of claims 1 to 5, wherein the catalyst contains transition metal ions and bromide ions in a ratio of 1:(from 0.01 to 2), in particular 1:(from 0.1 to 1) and preferably 1:(from 0.2 to 0.7).

7. The process as claimed in one or more of claims 1 to 6, wherein the catalyst contains cobalt ions and manganese ions in a ratio of 1:(from 0.01 to 3), in particular 1:(from 0.05 to 1).

8. The process as claimed in one or more of claims 1 to 7, wherein the catalyst is employed in a quantity corresponding to from 0.002 to 0.1 mol, in particular from 0.003 to 0.03 mol and preferably from 0.005 to 0.015 mol of transition metal ions per mole of 5-fluoro-2-bromotoluene.

9. The process as claimed in one or more of claims 1 to 8, wherein the acidic solvent employed is an aliphatic carboxylic acid having 1 to 8 carbon atoms, in particular 2 to 6 carbon atoms.

10. The process as claimed in one or more of claims 1 to 9, wherein the acidic solvent employed is acetic acid, propionic acid, n-butyric acid, isobutyric acid or mixtures thereof.

11. The process as claimed in one or more of claims 1 to 10, wherein the acidic solvent employed is acetic acid and/or propionic acid, especially acetic acid.

12. The process as claimed in one or more of claims 1 to 11, wherein the acidic solvent is employed in anhydrous form.

13. The process as claimed in one or more of claims 1 to 12, wherein during the reaction with oxygen or an oxygen-containing gas the reaction mixture does not contain more than 15% by weight of water.

14. The process as claimed in one or more of claims 1 to 13, wherein during the reaction with oxygen or an oxygen-containing gas the reaction mixture does not contain more than 5% by weight of water.

15. The process as claimed in one or more of claims 1 to 14, wherein the oxygen-containing gas employed is air, especially dry air.

16. The process as claimed in one or more of claims 1 to 15, wherein the reaction with oxygen or an oxygen-containing gas is carried out at from 130 to 180°C, in particular from 135 to 160°C.

17. The process as claimed in one or more of claims 1 to 16, wherein an oxygen partial pressure of at least 0.15 MPa, measured at the point of entry into the liquid phase, is applied.

18. The process as claimed in one or more of claims 1 to 17, wherein an oxygen partial pressure of at least 0.25 MPa, in particular 0.4 MPa, measured at the point of entry into the liquid phase, is applied.

19. The process as claimed in one or more of claims 1 to 18, wherein the 5-fluoro-2-bromobenzoic acid formed is separated off by filtration, and the resulting filtrate is freed from water by distillation and reused as the reaction medium.

20. The process as claimed in one or more of claims 1 to 19, wherein the 5-fluoro-2-bromobenzoic acid is reacted with an aqueous ammonia solution.

21. The process as claimed in one or more of claims 1 to 20, wherein the 5-fluoro-2-bromobenzoic acid is reacted with an aqueous ammonia-containing solution whose concentration is from 20 to 50% by weight, in particular from 25 to 33% by weight.

22. The process as claimed in one or more of claims 1 to 21, wherein the reaction with ammonia is carried out at from 90 to 150°C, in particular from 100 to 130°C.

23. The process as claimed in one or more of claims 1 to 22, wherein the reaction with ammonia is carried out at a pressure of from 0.2 to 2.5 MPa, in particular from 0.3 to 1.5 MPa and preferably from 0.5 to 1.2 MPa.

24. The process as claimed in one or more of claims 1 to 23, wherein 5-fluoro-2-bromobenzoic acid is passed continuously into an ammonia solution, in the form of a solution of an ammonium salt and/or alkali metal salt of the 5-fluoro-2-bromobenzoic acid in water.

25. The process as claimed in one or more of claims 1 to 24, wherein an acidic solution of 5-fluoroanthranilic acid in water is treated with active charcoal, the active charcoal is filtered off, and alkali is added to the filtrate in order to precipitate 5-fluoroanthranilic acid.

## Revendications

1. Procédé pour la préparation de l'acide 5-fluoranthranilique caractérisé en ce que l'on fait réagir le 5-fluoro-2-bromotoluène, en présence d'un catalyseur et d'un solvant acide, avec l'oxygène ou un gaz contenant de l'oxygène, à 80 à 220 °C, on sépare l'acide 5-fluoro-2-bromobenzoïque formé et on fait réagir avec l'ammoniac à 70 à 180 °C.

2. Procédé selon la revendication 1, caractérisé en ce que le catalyseur contient des ions de métaux de transition.

3. Procédé selon la revendication 1 ou 2, caractérisé en ce que le catalyseur contient des ions cobalt.

4. Procédé selon une ou plusieurs des revendications 1 à 3, caractérisé en ce que le catalyseur contient des ions cobalt et manganèse.

5. Procédé selon une ou plusieurs des revendications 1 à 4, caractérisé en ce que le catalyseur contient de plus des ions de métaux de transition des ions bromure.

6. Procédé selon une ou plusieurs des revendications 1 à 5, caractérisé en ce que le catalyseur contient des ions de métaux de transition et des ions bromure dans un rapport de 1:(0,01 à 2), notamment de 1:(0,1 à 1), de préférence de 1:(0,2 à 0,7).

7. Procédé selon une ou plusieurs des revendications 1 à 6, caractérisé en ce que le catalyseur contient des ions cobalt et des ions manganèse dans un rapport de 1:(0,01 à 3) notamment de 1:(0,05 à 1).

8. Procédé selon une ou plusieurs des revendications 1 à 7, caractérisé en ce que l'on ajoute au catalyseur une quantité d'ions de métaux de transition correspondant à 0,002 à 0,1, plus particulièrement à 0,003 à 0,03, de manière préférée à 0,005 à 0,015 mole par rapport à 1 mole de 5-fluoro-2-bromotoluène.

9. Procédé selon une ou plusieurs des revendications 1 à 8, caractérisé en ce que l'on utilise comme solvant acide un acide carboxylique aliphatique comportant de 1 à 8, plus particulièrement de 2 à 6 atomes de carbone.

10. Procédé selon une ou plusieurs des revendications 1 à 9, caractérisé en ce que l'on utilise comme solvant acide l'acide acétique, l'acide propionique, l'acide n-butyrique ou l'acide i-butyrique, ou leurs mélanges.

11. Procédé selon une ou plusieurs des revendications 1 à 10, caractérisé en ce que l'on utilise comme solvant acide, l'acide acétique et/ou l'acide propionique, plus particulièrement l'acide acétique.

12. Procédé selon une ou plusieurs des revendications 1 à 11, caractérisé en ce que l'on utilise le solvant acide sous forme anhydre.

13. Procédé selon une ou plusieurs des revendications 1 à 12, caractérisé en ce que le mélange réactionnel au cours de la réaction avec l'oxygène ou un gaz contenant de l'oxygène ne contienne pas plus de 15 % en poids d'eau.

14. Procédé selon une ou plusieurs des revendications 1 à 13, caractérisé en ce que le mélange réactionnel au cours de la réaction avec l'oxygène ou un gaz contenant de l'oxygène ne contient pas plus de 5 % en poids d'eau.

15. Procédé selon une ou plusieurs des revendications 1 à 14, caractérisé en ce que l'on utilise l'air comme gaz contenant de l'oxygène, plus particulièrement l'air sec.

16. Procédé selon une ou plusieurs des revendications 1 à 15, caractérisé en ce que l'on met en oeuvre la réaction avec l'oxygène ou un gaz contenant de l'oxygène à une température de 130 à 180, plus particulièrement de 135 à 160 °C.

17. Procédé selon une ou plusieurs des revendications 1 à 16, caractérisé en ce que l'on utilise une pression partielle d'oxygène d'au moins 0,15 MPa mesurée à l'entrée dans la phase liquide.

18. Procédé selon une ou plusieurs des revendications 1 à 17, caractérisé en ce que l'on utilise une pression partielle d'oxygène d'au moins 0,25, plus particulièrement de 0,4 MPa mesurée à l'entrée dans la phase liquide.

19. Procédé selon une ou plusieurs des revendications 1 à 18, caractérisé en ce que l'on sépare par filtration l'acide 5-fluoro-2-bromobenzoïque formé, on débarrasse le filtrat de l'eau par distillation et on l'utilise à nouveau comme milieu réactionnel.

20. Procédé selon une ou plusieurs des revendications 1 à 19, caractérisé en ce que l'on fait réagir l'acide 5-fluoro-2-bromobenzoïque avec une solution aqueuse d'ammoniac.

21. Procédé selon une ou plusieurs des revendications 1 à 20, caractérisé en ce que l'on fait réagir l'acide 5-fluoro-2-bromobenzoïque avec une solution aqueuse d'ammoniac ayant une teneur de 20 à 50, plus particulièrement de 25 à 33 % en ammoniac.

22. Procédé selon une ou plusieurs des revendications 1 à 21, caractérisé en ce que l'on met en oeuvre la réaction avec l'ammoniac à une température de 90 à 150, plus particulièrement de 100 à 130 °C.

23. Procédé selon une ou plusieurs des revendications 1 à 22, caractérisé en ce que l'on met en oeuvre la réaction avec l'ammoniac à une pression de 0,2 à 2,5, notamment de 0,3 à 1,5 MPa, de préférence de 0,5 à 1,2 MPa.

24. Procédé selon une ou plusieurs des revendications 1 à 23, caractérisé en ce que l'on amène, en continu, à une solution d'ammoniac, l'acide 5-fluoro-2-bromobenzoïque sous forme de solution d'un sel d'ammonium ou d'un sel de métal alcalin de l'acide 5-fluoro-2-bromobenzoïque dans l'eau.

25. Procédé selon une ou plusieurs des revendications 1 à 24, caractérisé en ce que l'on traite une solution acide de l'acide 5-fluoranthranilique dans l'eau avec du charbon actif, on sépare par filtration le charbon actif et on fait précipiter l'acide 5-fluoranthranilique dans le filtrat en ajoutant un alcali.
